# EUROPÄISCHE PATENTSCHRIFT

(11) **EP 2 070 912 B1**
(45) Veröffentlichungstag und Bekanntmachung des Hinweises auf die Patenterteilung: **12.09.2012**
(21) Anmeldenummer: 07122809.2
(22) Anmeldetag: 11.12.2007
(51) Int. Cl.: C07D 207/267

(54) **Verfahren zur Vinylierung von Amiden**
Method for the vinylation of amides
Procédé de vinylation d'amides

(43) Veröffentlichungstag der Anmeldung: 17.06.2009
(73) Patentinhaber: BASF SE, 67056 Ludwigshafen (DE)
(72) Erfinder: Vogelsang, Regina, 67063 Ludwighafen (DE); Käshammer, Stefan Dr., 67105 Schifferstadt (DE); Eiden, Ulrich, Dr., 67271 Kindenheim (DE); Brand, Alexandra Dr., 64367 Mühltal (DE); Tuttelberg, Lembit, 68305 Mannheim (DE); Staffel, Wolfgang, Dr., 67165 Waldsee (DE)

(56) Entgegenhaltungen:
- EP-A- 0 732 324
- DE-A1- 3 215 093
- US-A- 4 873 336

## Beschreibung

Die Erfindung betrifft ein Verfahren zur Herstellung einer N-Vinylverbindung ausgewählt aus cyclischen N-Vinylamiden und nicht cyclischen Vinylamiden der Formel I 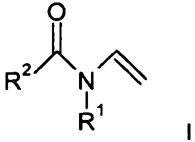 worin R1 für ein H Atom oder eine C1 bis C4 Alkylgruppe und R2 für eine C1 bis C10 Alkylgruppe stehen, durch Vinylierung einer Verbindung mit mindestens einem Stickstoffatom, im nachfolgenden kurz als Verbindung bezeichnet, mit Acetylen, welches dadurch gekennzeichnet, dass die Verbindung vor der Vinylierung in einer Reaktionszone mit einem Alkalihydroxid umgesetzt wird und die mittlere Verweilzeit des Alkalihydroxids und der Verbindung in der Reaktionszone kleiner 6 Minuten ist.

Technisch von besonderer Bedeutung ist N-Vinylpyrrolidon, welches durch Vinylierung von Pyrrolidon mit Acetylen hergestellt werden kann. Aus DE-A 195 09 362 ist bekannt, Pyrrolidon in einem ersten Verfahrensschritt mit einem Alkalihydroxid umzusetzen und die anschließende Vinylierung mit dem Reaktionsprodukt dieser Umsetzung durchzuführen.

Bei der Umsetzung von Pyrrolidon mit einem Alkalihydroxid entsteht ein Pyrrolidat, welches die nachfolgende Vinylierung mit Acetylen katalysiert. Nachteilig ist, dass darüber hinaus auch Nebenprodukte entstehen. Insbesondere kommt es zur Öffnung des Fünfrings und Bildung von Aminobuttersäure. Die Nebenprodukte vermindern den Anteil des Pyrrolidons, der für die Vinylierung zur Verfügung steht, und somit auch die Gesamtausbeute des Verfahrens.

Gewünscht ist ein Verfahren, bei dem der Anteil von Nebenprodukten möglichst gering ist. Gleichzeitig soll die Raumzeitausbeute möglichst gut sein, insbesondere sollen sich Maßnahmen zur Verringerung der Nebenproduktbildung nicht nachteilig auf die Aktivität des Katalysators auswirken.

Demgemäß wurde das oben definierte Verfahren gefunden.

Das erfindungsgemäße Verfahren eignet sich zur Herstellung der obigen N-Vinylamide. Die N-Vinylamide können cyclisch (N-Vinyllactame) sein. Bevorzugte N-Vinyllactame sind N-Vinylcaprolactam, N-Vinylpiperidon und N-Vinylpyrrolidon. Nicht cyclische Vinylamide sind solche der Formel 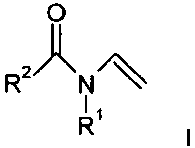 worin R1 für ein H Atom oder eine C1 bis C4 Alkylgruppe und R2 für eine C1 bis C10 Alkylgruppe stehen.

Ein bevorzugtes nicht-cyclisches N-Vinylamid ist N-Vinyl-N-methylacetamid (VIMA) mit der Formel

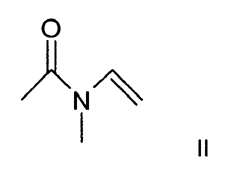

Ausgangsprodukt für das Verfahren sind Verbindungen mit mindestens einem Stickstoffatom (kurz Verbindung). Entsprechend den vorstehenden Produkten handelt es sich um Amide, seien es cyclische Amide (Lactame), wie Caprolactam, Piperidon oder Pyrrolidon oder nicht-cyclische Amide, wie Acetamid oder N-Methylacetamid.

In einer besonders bevorzugten Ausführungsform handelt es sich bei der Verbindung um Pyrrolidon und bei der daraus erhaltenen N-Vinylverbindung um N-Vinylpyrrolidon.

In einer weiteren bevorzugten Ausführungsform handelt es sich bei der Verbindung um N-Metylacetamid und bei der N-Vinylverbindung um N-Vinyl-N-methylacetamid (VIMA).

Die Verbindung wird erfindungsgemäß zunächst mit einem Alkalihydroxid umgesetzt. Es kann sich z. B. um ein Lithium-, Natrium- oder Kaliumhydroxid handeln; besonders bevorzugt ist Kaliumhydroxid.

Das Alkalihydroxid wird vorzugsweise in Form einer wässrigen Lösung verwendet. Der Gehalt an Alkalihydroxid kann z. B. 5 bis 90 Gew. %, bezogen auf die Lösung betragen; insbesondere beträgt er 30 bis 60 Gew. %, besonders bevorzugt 45 bis 55 Gew. %.

Die Umsetzung der Verbindung mit dem Alkalihydroxid erfolgt vorzugsweise bei Temperaturen von 50 bis 250 °C und 1 mbar bis 1 bar, insbesondere bei 20 bis 250 °C. Die Temperatur am Kolonnenkopf beträgt vorzugsweise 20 bis 100, insbesondere 25 bis 60 °C; die Temperatur im Kolonnensumpf beträgt vorzugsweise 100 bis 250 °C, insbesondere 120 bis 200°C.

Das Verfahren wird vorzugsweise semi-kontinuierlich oder kontinuierliche betrieben. Besonders bevorzugt wird es kontinuierlich betrieben.

Vorzugsweise erfolgt die Umsetzung in einer Kolonne, besonders bevorzugt in einer Füllkörperkolonne oder Packungskolonne, welche bei den vorstehenden Temperaturen und Drucken betrieben wird. Sie wird vorzugsweise kontinuierlich betrieben.

Geeignet sind auch Kolonnen, die sowohl Füllkörper als auch Packungen enthalten, z. B. im unteren Teil Füllkörperschüttungen enthalten und im oberen Teil mit Packungselementen (z. B. eingebaute Stahlbleche) aufweisen.

Die Kolonne hat vorzugsweise mindestens zwei, besonders bevorzugt mindestens 3 theoretische Böden. Sie kann z. B. 2 bis 100, insbesondere 3 bis 20 theoretische Böden haben.

Das Alkalihydroxid und die Verbindung werden vorzugsweise im oberen Drittel, besonders bevorzugt im oberen Viertel der Kolonne zugegeben.

Die mittlere Verweilzeit des Alkalihydroxids und der Verbindung in der Reaktionszone, bzw. Kolonne, ist kleiner 6 Minuten, insbesondere kleiner 5 Minuten, besonders bevorzugt beträgt sie 50 bis 200 Sekunden.

Bei der Umsetzung bildet sich das entsprechende Alkalisalz der Verbindung, z.B. im Falle des Pyrrolidons das Pyrrolidat, bzw. Kaliumpyrrolidat.

Die Menge an Alkalihydroxid wird vorzugsweise so gewählt, dass 0,25 bis 25 Gew. %, vorzugsweise 1 bis 10 Gew. % der Verbindung als Alkalisalz (Pyrrolidon als Pyrrolidat, bzw. Kaliumpyrrolidat) vorliegt.

Das Produkt der Umsetzung kann im unteren Teil der Kolonne oder am Kolonnensumpf kontinuierlich abgezogen werden. Anschließend erfolgt dann vorzugsweise in einem separaten Reaktionsgefäß die Umsetzung der Verbindung mit Acetylen.

Das Alkalisalz katalysiert die anschließende Umsetzung mit Acetylen (Vinylierung). Der Austrag aus der vorstehenden Kolonne kann mit weiterer Verbindung (Pyrrolidon) gemischt werden.

Die für die Vinylierung verwendete Verbindung (Pyrrolidon) kann dann insbesondere 0,25 bis 10 Gew. %, insbesondere 1,5 bis 6 Gew. % Pyrrolidat enthalten.

Durch das erfindungsgemäße Verfahren sind die N- Vinylamide, z.B. N-Vinylpyrrolidon in hoher Ausbeute und Reinheit erhältlich. Insbesondere ist der Anteil von Nebenprodukten, z.B. im Falle des N-Vinylpyrrolidons des Nebenprodukts Aminobuttersäure und der Nebenprodukte, welche sich durch Umsetzung von Aminobuttersäure mit Acetylen ergeben, deutlich verringert.

Das erhaltene N-Vinylamid, insbesondere das N-Vinylpyrrolidon, kann ggf. durch Destillation abgetrennt werden und kann im Allgemeinen mit einer Reinheit von mehr als 99 Gew. %, besonders bevorzugt von mehr als 99, 5 Gew. % gewonnen werden. Die Raumzeitausbeute des Gesamtverfahrens, einschließlich der anschließenden Vinylierung, ist weiterhin sehr gut. Die kurze Verweilzeit bei der Umsetzung der Verbindung mit dem Alkalihydroxid wirkt sich demnach nicht nachteilig auf die katalytische Aktivität aus.

### Beispiele

### Vergleichsbeispiel 1

2-Pyrrolidon und 50 %ige KOH-Lösung in Wasser wurden vorgelegt und im Vakuum innerhalb einer Stunde (Verweilzeit) das Wasser und entstehendes Wasser abdestilliert. In dem so hergestellten Reaktionsansatz wurden Aminobuttersäure sowie Oligomere und Hydrolyseprodukte in nennenswerten Mengen gefunden.

### Beispiel 2

2-Pyrrolidon und eine 48 Gew. % ige KOH-Lösung in Wasser wurde in den Flüssigkeitsverteiler einer Füllkörperkolonne gegeben und das Wasser aus der KOH und das entstehende Wasser in dieser Kolonne abgetrennt. Die Verweilzeit in der Reaktionszone betrug unter 100 Sekunden.

Aminobuttersäure und deren Folgeprodukte konnten im Reaktionsprodukt nicht nachgewiesen werden.

## Patentansprüche

1. Verfahren zur Herstellung einer N-Vinylverbindung ausgewählt aus cyclischen N-Vinylamiden und nicht cyclischen Vinylamiden der Formel I 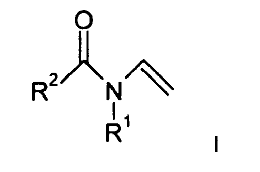 worin R1 für ein H Atom oder eine C1 bis C4 Alkylgruppe und R2 für eine C1 bis C10 Alkylgruppe stehen,
durch Vinylierung einer Verbindung mit mindestens einem Stickstoffatom, im nachfolgenden kurz als Verbindung bezeichnet, mit Acetylen, **dadurch gekennzeichnet, dass**
- die Verbindung vor der Vinylierung in einer Reaktionszone mit einem Alkalihydroxid umgesetzt wird und
- die mittlere Verweilzeit des Alkalihydroxids und der Verbindung in der Reaktionszone kleiner 6 Minuten ist.

2. Verfahren gemäß Anspruch 1, **dadurch gekennzeichnet, dass** es sich bei der N-Vinylverbindung um N-Vinyl-N-methylacetamid oder N-Vinylpyrrolidon handelt.

3. Verfahren gemäß einem der Ansprüche 1 oder 2, **dadurch gekennzeichnet, dass** es sich bei dem Alkalihydroxid um Kaliumhydroxid handelt.

4. Verfahren gemäß einem der Ansprüche 1 bis 3, **dadurch gekennzeichnet, dass** das Alkalihydroxid in Form einer wässrigen Lösung verwendet wird.

5. Verfahren gemäß einem der Ansprüche 1 bis 4, **dadurch gekennzeichnet, dass** die Umsetzung in einer Kolonne (Reaktionszone) erfolgt.

6. Verfahren gemäß Anspruch 5, **dadurch gekennzeichnet, dass** es sich um eine Füllkörperkolonne mit mindestens 2 theoretischen Böden handelt.

7. Verfahren gemäß Anspruch 5 oder 6, **dadurch gekennzeichnet, dass** das Alkalihydroxid und die Verbindung im oberen Drittel der Kolonne zugegeben werden.

8. Verfahren gemäß einem der Ansprüche 5 bis 7, **dadurch gekennzeichnet, dass** die Kolonne bei einem Druck von 1 mbar bis 1 bar und einer Temperatur von 20 bis 250°C betrieben wird.

9. Verfahren gemäß einem der Ansprüche 1 bis 8, **dadurch gekennzeichnet, dass** die mittlere Verweilzeit des Alkalihydroxids und der Verbindung in der Reaktionszone 50 bis 200 Sekunden beträgt.

10. Verfahren gemäß einem der Ansprüche 1 bis 9, **dadurch gekennzeichnet, dass** die Vinylierung der Verbindung in einem anschließenden Verfahrensschritt erfolgt.

## Claims

1. A process for preparing an N-vinyl compound selected from cyclic N-vinylamides and acyclic vinylamides of the formula I 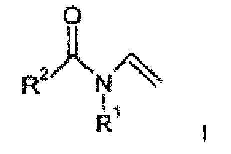 in which R1 is a hydrogen atom or a C1 to c4 alkyl group and R2 is a C1 to C10 alkyl group,
by vinylating a compound having at least one nitrogen atom, referred to hereinafter as compound for short, with acetylene, wherein
- before the vinylation, the compound is reacted with an alkali metal hydroxide in a reaction zone and
- the mean residence time of the alkali metal hydroxide and of the compound in the reaction zone is less than 6 minutes.

2. The process according to claim 1, wherein the N-vinyl compound is N-vinyl-N-methylacetamide or N-vinylpyrrolidone.

3. The process according to either of claims 1 and 2, wherein the alkali metal hydroxide is potassium hydroxide.

4. The process according to any of claims 1 to 3, wherein the alkali metal hydroxide is used in the form of an aqueous solution.

5. The process according to any of claims 1 to 4, wherein the reaction is effected in a column (reaction zone).

6. The process according to claim 5, wherein the column has random packing and has at least 2 theoretical plates.

7. The process according to claim 5 or 6, wherein the alkali metal hydroxide and the compound are added in the upper third of the column.

8. The process according to any of claims 5 to 7, wherein the column is operated at a pressure of from 1 mbar to 1 bar and a temperature of from 20 to 250°C.

9. The process according to any of claims 1 to 8, wherein the mean residence time of the alkali metal hydroxide and of the compound in the reaction zone is from 50 to 200 seconds.

10. The process according to any of claims 1 to 9, wherein the vinylation of the compound is effected in a subsequent process step.

## Revendications

1. Procédé de fabrication d'un composé de N-vinyle choisi parmi les N-vinylamides cycliques et les vinylamides non cycliques de formule I 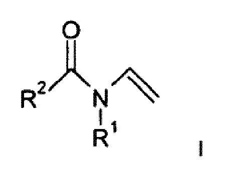 dans laquelle R1 représente un atome H ou un groupe alkyle en C1 à C4 et R2 représente un groupe alkyle en C1 à C10,
par vinylation d'un composé contenant au moins un atome d'azote, par la suite nommé composé en abrégé, avec de l'acétylène, **caractérisé en ce que**
- le composé est mis en réaction avec un hydroxyde alcalin dans une zone de réaction avant la vinylation et
- le temps de séjour moyen de l'hydroxyde alcalin et du composé dans la zone de réaction est inférieur à 6 minutes.

2. Procédé selon la revendication 1, **caractérisé en ce que** le composé de N-vinyle est le N-vinyl-N-méthylacétamide ou la N-vinylpyrrolidone.

3. Procédé selon l'une quelconque des revendications 1 ou 2, **caractérisé en ce que** l'hydroxyde alcalin est l'hydroxyde de potassium.

4. Procédé selon l'une quelconque des revendications 1 à 3, **caractérisé en ce que** l'hydroxyde alcalin est utilisé sous la forme d'une solution aqueuse.

5. Procédé selon l'une quelconque des revendications 1 à 4, **caractérisé en ce que** la réaction a lieu dans une colonne (zone de réaction).

6. Procédé selon la revendication 5, **caractérisé en ce qu'**il s'agit d'une colonne à corps de remplissage contenant au moins 2 plateaux théoriques.

7. Procédé selon la revendication 5 ou 6, **caractérisé en ce que** l'hydroxyde alcalin et le composé sont introduits dans le tiers supérieur de la colonne.

8. Procédé selon l'une quelconque des revendications 5 à 7, **caractérisé en ce que** la colonne est exploitée à une pression de 1 mbar à 1 bar et à une température de 20 à 250 °C.

9. Procédé selon l'une quelconque des revendications 1 à 8, **caractérisé en ce que** le temps de séjour moyen de l'hydroxyde alcalin et du composé dans la zone de réaction est de 50 à 200 secondes.

10. Procédé selon l'une quelconque des revendications 1 à 9, **caractérisé en ce que** la vinylation du composé a lieu lors d'une étape de procédé ultérieure.
